# EUROPEAN PATENT SPECIFICATION

(11) **EP 1 224 462 B1**
(45) Date of publication and mention of the grant of the patent: **29.06.2005**
(21) Application number: 00972027.7
(22) Date of filing: 06.10.2000
(51) Int. Cl.: G01N 33/543, G01N 33/68, A61K 35/14, G01N 33/80

(54) **ISOAGGLUTININ-DEPLETED BLOOD COMPOSITIONS AND METHODS OF MAKING SAME**
VON ISOAGGLUTININ BEFREITE BLUTZUSAMMENSETZUNGEN UND VERFAHREN ZU IHRER HERSTELLUNG
COMPOSES SANGUINS DEMUNIS D'ISOAGGLUTININE ET LEURS PROCEDES DE FABRICATION

(30) Priority: 08.10.1999 US 415854
(43) Date of publication of application: 24.07.2002
(73) Proprietor: V.I. Technologies, Inc., Watertown, MA 02471 (US)
(72) Inventor: CHIN, Sing, N., New York, NY 10013 (US); HAMMOND, David, J., Cortland Manor, NY 10567 (US)
(74) Representative: Jump, Timothy John Simon
(86) International application number: PCT/US2000/027711
(87) International publication number: WO 2001/027623

(56) References cited:
- US-A- 4 664 913
- US-A- 5 541 294
- JUDD W JOHN ET AL: "Isohemagglutinin-depleted solvent detergent plasma: A "universal" viral-inactivated plasma for patients of any ABO blood type." BLOOD, vol. 94, no. 10 SUPPL. 1 PART 1, 15 November 1999 (1999-11-15), page 375a XP002165151 Forty-first Annual Meeting of the American Society of Hematology;New Orleans, Louisiana, USA; December 3-7, 1999 ISSN: 0006-4971

## Description

### FIELD OF THE INVENTION

The invention relates generally to methods of removing selected proteins from a complex sample of proteins and in particular to methods of removing isoagglutinins from blood-derived products.

### BACKGROUND OF THE INVENTION

Human blood can vary from individual to individual. Multiple blood groups based on differences between these individuals have been described. In general, the phrase "blood group" is used to identify any one of the numerous types into which an individual's blood may be classified, based upon the presence or absence of certain antigens located on the surface of the individual's erythrocytes (*i*.*e*., red blood cells). Each specific blood group contains, or may contain, antibodies within the serum which react against the cells of other groups. These antibodies are referred to as "blood group antibodies" or "isoagglutinins".

More than thirty systems of blood group characterization systems or groupings have been described. One of the most commonly used is the ABO grouping system. The major isoagglutinins in this system are the anti-A, anti-B and anti A,B antibodies, which are comprised primarily of the IgM and IgG immunoglobulin isotypes. It has been demonstrated that: (*i*) the serum of blood group A contains antibodies to antigen B; (*ii*) that of blood group B contains antibodies to antigen A; (*iii*) that of blood group AB possesses neither antibody; and (*iv*) serum from blood group O possesses both types of antibodies.

Blood group differences must be considered when blood is transfused from one individual to another. Transfusion reactions precipitated by incompatible blood group antibodies can result in agglutination of blood cells in the transfused host. In addition, transfusions between such incompatible individuals can fix and activate complement, thus inducing the destruction of erythrocytes (*i*.*e*., hemolysis). This can lead to the development of anemia and other, similar complications. The transfusion of incompatible blood can result in a serious medical emergency which can result in death. In order to avoid immunohemolysis and other related transfusion reactions, donor plasma type and recipient blood group type are cross-matched or typed and screened.

A blood-derived composition lacking hemolysis-causing isoagglutinins can potentially be administered without regard for the blood group of either the donor or the recipient. Consequently, methods have been described for removing isoagglutinins from blood. However, the currently available methods can result in inefficient removal of blood group isoagglutinins, and can require long incubation times, or require repeated separation steps. In addition, most immunoadsorption-based methods for removing isoagglutinins are intended for a single application. This can result in increased costs for preparing isoagglutinin-free blood.

### SUMMARY OF THE INVENTION

The invention is based in part on the discovery of a procedure that allows for the repeated use of immunoadsorbent resins for removing isoagglutinins from blood. Accordingly, in one aspect, the invention provides a method for selectively removing an isoagglutinin from a blood-derived composition. The method includes providing a resin linked to an antigen specific for the isoagglutinin and washing the resin with a regeneration buffer to form a regenerated resin. The regenerated resin is then contacted with the blood-derived composition under conditions allowing for formation of an isoagglutinin-antigen complex on the resin. The blood-derived composition is then separated from the isoagglutinin-antigen complex, thus removing the isoagglutinin from the composition. If desired, the resin-antigen can be rewashed with the regeneration buffer and a second blood-derived composition added.

In another aspect, the invention provides a method of regenerating a resin for removing an isoagglutinin from a blood-derived composition. The method includes providing a resin linked to an antigen-isoagglutinin complex; and washing the resin with a regenerating buffer under conditions sufficient to selectively remove the isoagglutinin from the antigen-isoagglutinin complex, thereby regenerating the resin. The washed resin can then be mixed with a blood-derived composition including the isoagglutinin under conditions sufficient to form a second antigen-isoagglutinin complex. The blood-derived composition is then separated from the second antigen-isoagglutinin complex, thereby separating the blood-derived composition from the isoagglutinin.

In a further aspect, the invention includes a composition for binding an isoagglutinin. The composition includes a polymethacrylate resin linked to an antigen specific for the isoagglutinin, wherein the antigen is linked to said resin by a carboxyl group on the resin.

The methods and composition described herein can be used to provide a variety of blood-derived compositions, e.g., solvent-detergent (SD) plasma for use as a "universal" SD-plasma, which can be administered (*i*.*e*., transfused) into *any* individual regardless of their specific blood group type is described. These methods are particularly suitable for A, B, and O blood group compositions and provide clinically acceptable levels of for anti-A, anti-B, and anti-A,B final isoagglutinin titers for transfusion into noncompatible patients.

The details of one or more embodiments of the invention are set forth in the accompanying description below. Although any methods and materials similar or equivalent to those described herein can be used in the practice or testing of the present invention, the preferred methods and materials are now described. Other features, objects, and advantages of the invention will be apparent from the description and from the claims. In the specification and the appended claims, the singular forms also include the plural unless the context clearly dictates otherwise. Unless defined otherwise, all technical and scientific terms used herein have the same meaning as commonly understood by one of ordinary skill in the art to which this invention belongs. All patents and publications cited in this specification are incorporated by reference.

### BRIEF DESCRIPTION OF THE DRAWINGS

FIG. 1 represents a schematic flow-chart illustrating the preparation of the tri-(n-butyl) phosphate (TNBP)/Triton X-100-treated, isoagglutinin-depleted plasma.
FIG. 2: represents a schematic flow-chart illustrating the preparation of the Group B trisaccharide-Toyopearl AF-Carboxy-650M conjugated resin.
FIG. 3: illustrates the titers of anti-B IgM and anti-B IgG immunoglobulins present in the pooled-plasma pass through fractions of different cycles following immunoselection with the Group B trisaccharide-Toyopearl AF-Carboxy-650M conjugated resin.

### DETAILED DESCRIPTION OF THE INVENTION

The present invention provides methods for removing isoagglutinins from blood products using separation systems that can be used repeatedly.

This isoagglutinin-depleted compositions purified according to these methods maintain all of the inherent characteristics of the original blood composition, *i*.*e*., the same coagulation factor levels, protein content, and protective antibody titers (*e*.*g*. to parvovirus and hepatitis A), but will possess markedly reduced anti-A and/or anti-B isoagglutinin titers. These low levels of isoagglutinins allow for non-blood group type-specific or "universal" clinical transfusion use.

In general, a blood composition is passed through columns that contain a reusable resin to which are coupled chemically synthesized carbohydrate moieties that bind to and remove anti-A, anti-B, and/or anti-A,B antibodies (the isoagglutinins).

The method can be used on any blood-derived composition known to contain, or suspected of containing, one or more isoagglutinins. As used herein, the terms "blood-derived compositions" and "blood compositions" are used interchangeably and are meant to include whole blood, blood plasma, blood plasma fractions, blood plasma precipitate (*e*.*g*., cryoprecipitate, ethanol precipitate or polyethylene glycol precipitate), blood plasma supernatant (*e*.*g*., cryosupernatant, ethanol supernatant or polyethylene glycol supernatant), solvent/detergent (SD) plasma, platelets, intravenous immunoglobulin (IVIG), IgM, purified coagulation factor concentrate, fibrinogen concentrate, or various other compositions which are derived from human or animal. Blood-derived compositions also include purified coagulation factor concentrates (*e*.*g*., factor VIII concentrate, factor IX concentrate, fibrinogen concentrate, and the like) prepared by any of various methods common in the art including ion exchange, affinity, gel permeation, and/or hydrophobic chromatography or by differential precipitation.

Isoagglutinins which can be removed include, *e.g*., anti-A isoagglutinins, anti-B isoagglutinins, anti-A,B isoagglutinins. The blood composition containing, or suspected of containing, isoagglutinins is mixed with a resin to which an antigen specific for at least one of the isoagglutinins is linked. Resin-antigen combinations are well-known in the art. See, *e*.*g*., U.S. Patent Nos. 4,362,720; 4,308,376; 4,238,473; 4,195,174; 4,137,401, 4,664,913, and 5,541,294. There are many examples of resins used for affinity chromatography-based isoagglutinin removal, See United States Patents 5,541,294 and 4,664,913, incorporated herein by reference. Important factors in selecting the resin include minimal non-specific adsorption of protein (*e.g*., anti-virus antibodies and coagulation factors) and the ability to be derivatized with the antigen of choice. A preferred resin for use is one that contains a polymethacrylate matrix. The most preferred is Toyopearl AF-Carboxy resin (Toyo-Haas, Japan). Additionally, the Toyopearl AF-Carboxy resin has a carboxyl group that can be used to couple ligands possessing a free amine group. The resin is also amenable to production-scale chromatography.

Preferred antigens are those that are specific for blood group antibodies. Particularly preferred antigens include oligosaccharides, *e.g*., saccharides having 3, 4, 5, 6, 7, 8, 9, 10, 11, or 12 or more saccharides that mimic the binding of the entire antigen to the blood group antibodies. For the B-Resin (*i*.*e*., B-trisaccharide), the preferred density of the ligand ranges from 0.8 to 1.5 µmol/ml, with the most preferred density being 1.0 µmol/ml. For the A-Resin (*i.e*., A-trisaccharide), the preferred density of the ligand is 1.2 µmol/ml.

Prior to purification of the blood product, the resin-antigen combination is blocked by treatment with ethanolamine. The preferred plasma resin-contact time ranges from two to six minutes, and is preferably four minutes, and the preferred plasma volume per resin volume should ranges from 20:1 to 40:1, and most preferably is 30:1. The resin can stored prior to use in an ethanol/water solution at low temperature, *e.g*., 20% ethanol at 4 °C.

The antigen is attached to the resin by any means that is stable enough to allow for multiple uses of the column without loss of the antigen ligand. The linkage can be covalent, or a stable non-covalent interaction. An amide linkage is preferred for the antigen-resin complex. Typically, a trisaccharide having a free terminal amine is coupled with a carboxyl functionality of the Toyopearl resin.

Contact of an antigen with its corresponding isoagglutinin in the blood-derived composition results in the formation of a antigen-isoagglutinin complex. The complex is retained on the antigen-resin, while substantially all of the remainder of the blood components is not bound by the antigen or resin Thus, the antigen-resin separates the isoagglutinin from the blood product composition.

The resin-antigens can be used as part of other separation technologies, *e*.*g*., chromatographic separation and batch absorption. When chromatography is used, a resin-antigen is equilibrated in a buffer (*e*.*g*., phosphate buffered saline [PBS] or saline) with a pH in the range of approximately 2 to 10, with a preferred range of 3.5 to 9.5, and with a most preferred pH range of 4 to 7.8. The equilibration temperature may range from below 0 °C to 45 °C, with a preferred temperature ranging from 19 °C to 26 °C, and a most preferred temperature of 23 °C. A blood-derived composition containing blood group antibodies and other blood components, e.g., coagulation factors, is then run through the resin column with a contact time ranging from less than 1 to 120 minutes, with 2 to 10 minutes, *e.g*., 4 minutes, being the preferred contact time. The retention of coagulation factors can be influenced by the specific resin selected, and more particularly the matrix of the resin. Preferably, the resin-antigen combinations remove isoagglutinins without substantially removing coagulation factors.

Batch absorption is another affinity-based technique which may be used in addition to, or instead of, column chromatography. In batch adsorption, an antigen- resin is added to a composition containing blood group antibodies and coagulation factors in a suitable container (*e.g.,* an inert polymer), mixed at a suitable temperature, *e.g*., 0 °C to 45 °C (with a preferred temperature being ambient) for a period of at least 1 hour (with the preferred period being 4 hours). The blood composition, now substantially free of the isoagglutinin, is then removed from the resin-antigen complex by art-recognized techniques such as gravity sedimentation, filtration, or centrifugation.

The resin-antigen complex is then washed with a regeneration buffer or agent.. The regeneration buffer removes isoagglutinins which have bound to the antigen-resin, and renders the antigen available for binding to a second isoagglutinin, *e*.*g*., an isoagglutinin present in a second blood composition or blood-derived composition subsequently added to the antigen-resin column. It is preferred that the regeneration buffer maintain the structural and functional integrity of both the antigen and the resin. Thus, removal of the bound isoagglutinins with a regeneration buffer permits reuse of the resin-antigen combination.

It has been unexpectedly found that sodium thiosulfate is a strong regeneration buffer or agent. Accordingly, regeneration buffers can include sodium thiosulfate from, *e*.*g*., 0.5M up to a saturated solution at room temperature. A preferred concentration of sodium thiosulfate is 3.0M. While not wishing to be bound by theory, it is believed that sodium thiosulfate acts, in part, as an oxidizing agent for the desorption of antibodies from the affinity resin. This mechanism may involve the reduction of disulfhydryl groups (-S-S-) to -SH groups between interchain F_{ab} immunoglobulin fragments and/or between the light and heavy chain subunits, thus causing a more complete dissociation of the antibody from the antibody-antigen complex. Another possibility may be that the chaotophic properties of sodium thiosulfate solubilize isoagglutinins bound to the resin-antigen.

The regeneration buffer or agent may be used in conjunction with other agents, such as urea, ethanol, acetic acid, and peracetic acid. The use of sodium thiosulfate in combination with urea and ethanol (EtOH) washes serves not only to regenerate the column for further use, but also inactivates and removes virus and prion contaminants. Peracetic acid further sanitizes the resin-antigen combination without damaging either resin or the antigen. The components of the regeneration buffer, *e*.*g*., sodium thiosulfate, urea, ethanol, or acetic acid may be used sequentially, simultaneously, or in various combinations thereof.

Regeneration of columns containing a resin-saccharide antigen using a regeneration agent allows for the columns to be re-used multiple times, e.g., 2, 5, 10, 15,25, 50,75, or 100 or more times, without a decrease in binding of the selected isoagglutinin, or an increase in non-specific binding of other proteins and coagulation factors indigenous to the blood composition. The resin can be used and regenerated for reuse on the order of 2, 5, 10, 15, 25, 50, 75, 100 or 200 or more times.

Previously described assays can be used to quantitate the removal of isoagglutinins and to ascertain the amount of non-isoagglutinin proteins, *e*.*g*., blood coagulation factors, lost to the resin-antigen complex following each regeneration. To determine whether the blood group-specific isoagglutinins have been removed from a blood-derived composition, the blood group antibody titer within the composition is determined after contacting the composition with a resin-antigen combination. This may be determined by either the immediate spin test (IS), the direct blood group antibody test (DAT) or the indirect Coombs test (ICT). In order to determine the amount of coagulation factors which were removed from the blood-derived compositions, the treated blood-derived compositions may be assayed for activities of the coagulation factor or factors of interest (*e*.*g*., factors V, VII, VIII, IX, X, XI, XII, and XIII), by a one-step clotting method which determines the degree of correction in the clotting time of a plasma deficient in the particular factor(s) in the presence of activated partial thromboplastin (APTT) reagent, or by various other assay methods known within the art. For example, isoagglutinin levels in blood compositions can be assayed using the MTS Anti-IgG Gel Card System™ and the MTS Buffered Gel Card System™ available from Ortho Diagnostics.

To evaluate the protein content and distribution of the blood-derived compositions, protein content may be measured by Biuret reagent and protein distribution may be measured by SDS-polyacrylamide gel electrophoresis.

Virus-containing blood-derived compositions may also be inactivated (*i*.*e*., made non-infective) either before or after removing the blood group-specific antibodies from the virus-containing composition. Fresh frozen plasma (FFP) that has been treated with solvent and detergent to inactivate virus is referred to as solvent-detergent plasma (SD-plasma). Various methods of viral inactivation are discussed in U.S. Pat. No. 4,764,369, the disclosure of which is incorporated herein by reference, in its entirety. The removal of the chemicals utilized in viral-inactivation methods are discussed in U.S. Pat. No. 5,094,960, the disclosure of which is incorporated herein by reference, in its entirety.

Solvent-Detergent plasma is a plasma composition that has been treated with an organic solvent and a detergent to remove blood-borne lipid coated viruses. Many different reagents used to accomplish this have been proposed in the art. See, *e*.*g*., U.S. Patent Nos. 4,481,189; 4,540,573; 4,764,369; 4,789,545, all incorporated herein by reference. The most preferred reagents of this invention include an effective amount of an alkylphosphate reagent, preferably tri-(n-butyl) phosphate (TNBP) as the "solvent" and Triton X-100 as the detergent. Treatment of a sample with these reagents is followed by extraction with soybean oil followed by column chromatography on a standard hydrophobic resin to remove the virus-inactivating reagents. These methods provide viral-inactivated blood-derived compositions without denaturing the indigenous proteins.

Other viral inactivation methods may also be used (*e*.*g*., photodynamic inactivation of viruses in the presence of methylene blue or the thermal inactivation of viruses in the presence of sugars and/or amino acids). Blood group antibody removal may be performed at any time relative to virus-inactivation: prior to, during, or following inactivation procedure. Viruses which are inactivated by these methods include, *e.g*., vesicular stomatitis virus (VSV), sindbis virus, human immunodeficiency virus (HIV), hepatitis B virus and hepatitis C virus.

The following examples are presented in order to more fully illustrate the preferred embodiments of the invention. These examples should in no way be construed as limiting the scope of the invention, as defined by the appended claims.

### EXAMPLES

The present invention may utilize the A-trisaccharide and/or B-trisaccharide in the depletion of isoagglutinins from blood-based compositions, depending upon specific blood group type. However, the following sections, by way of example and not of limitation, will primarily discuss those methodologies and results utilizing the B-trisaccharide.

### EXAMPLE 1 Preparation of TNBP/Triton X-100-Treated (SD), Isoagglutinin-Depleted Plasma

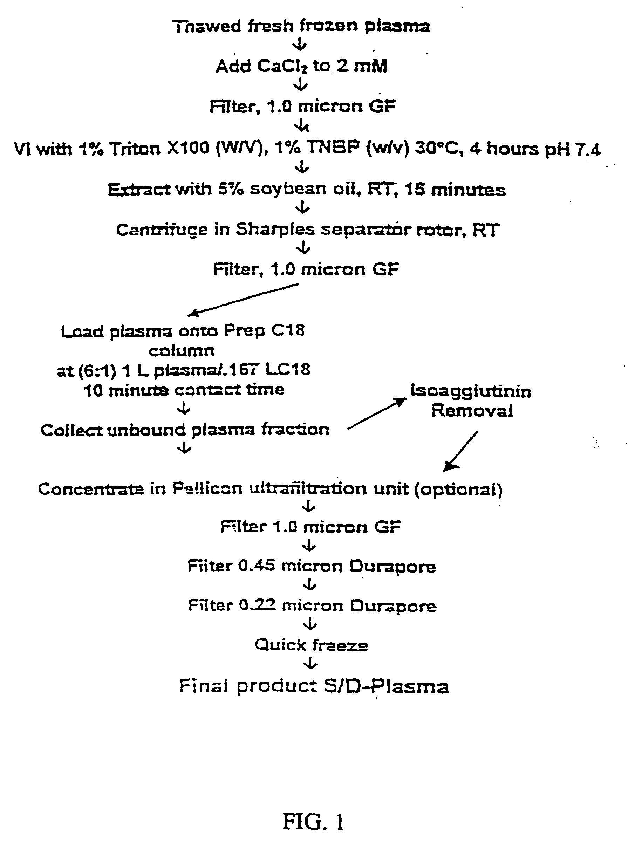

The present invention involves the incorporation of an isoagglutinin removal step, using a resin-antigen composition in the manufacture of SD-Plasma. FIG. 1 represents a schematic flow-chart illustrating the preparation of the tri-(n-butyl) phosphate (TNBP)/Triton X-100-treated, isoagglutinin-depleted plasma. This process is similar to the protocol known in the art for the production of Solvent-Detergent (SD) plasma, in which the plasma is treated with 1% tri (n-butyl) phosphate (TNBP) and 1% Triton X-100, at 30 °C for four hours

The modification of the SD protocol consists of the addition of an isoagglutinin-depletion/removal chromatography column after the C18 column, *i*.*e*., either before or after the Pellicon® ultrafiltration steps of the SD-Plasma production process (FIG. 1). In this modification, the eluate (or a concentrate of the eluate) from the C18 column is passed through the appropriate affinity column(s). For example, when starting with pooled type "A" plasma, the sample is passed through a column containing the B antigen (B-resin). Likewise, starting with pooled type "B" plasma, the sample is passed through a column containing the A antigen (A-resin). Samples of "O" plasma are passed through two affinity columns (A- and B-resins) placed in series immediately downstream from the C18 column. Plasma which is of type "AB" will require no specific isoagglutinin removal.

The removal of substantially all of the isoagglutinins which are responsible for the differences among the four commercially-available SD-Plasma types, allows for the production of a single type of SD-Plasma that would be suitable for administration to all recipients regardless of blood group type.

### EXAMPLE 2 Preparation of Blood Group-Specific Trisaccharide-Loaded Resins

Studies were performed in order to ascertain the optimal loading density of the trisaccharide ligand. In the first series of experiments, acceptable direct agglutination test (*i*.*e*., immediate spin test) and indirect Coombs test results were achieved at initial trisaccharide concentrations of 1 and 10 µmol/ml. As much as 200 ml of plasma could be applied to I ml of resin. However a 0.1 µmol/ml initial concentration failed to reduce isoagglutinin titers to the acceptable ≤1:2 value. In a second series of experiments, isoagglutinin titers ≤1:2 were obtained with all resins prepared using 0.75 to 3.0 µmol/ml B-trisaccharide. Although resins with higher trisaccharide density could successfully process larger volumes of plasma, the most practical implementation based upon resin cost is achieved with a I µmol/ml trisaccharide density. Similar results were obtained for the A-trisaccharide.

The structure of the A resin is: 2-O-(α-L-fuco-pyranosyl)-3-O-(2-acetamido-2-deoxy-α-D-galactopyranosyl)-β-D-galactopyranoside.

The structure of the B resin is: Galactose-α-1-3 (fucα1-2) Galβ1-O-.

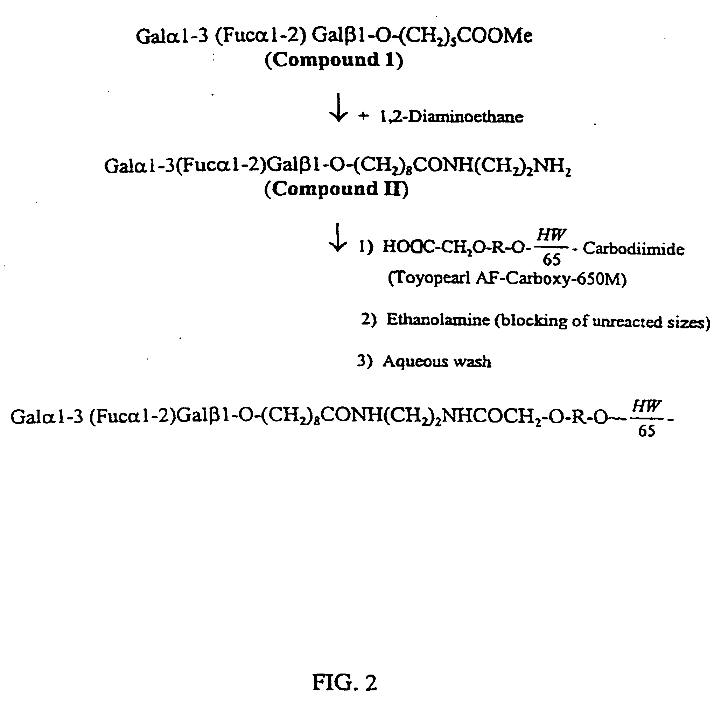

FIG. 2 represents a schematic flow-chart illustrating the preparation of Group B trisaccharide-Toyopearl AF-Carboxy-650M conjugated resin. Compound I (162 mg) was dissolved in 10 ml methanol and 5 ml of 1,2-diaminoethane added. After 4 days at ambient temperature the mixture was poured into 200 ml of xylene and rotary-evaporated with a water bath temperature < 40 °C until a gummy solid remained. At that time an additional 200 ml of xylene was added, and again the mixture was rotary-evaporated to dryness. Following evaporation, the product was subjected to a vacuum for 30 minutes to complete the drying process.

A total of 10 g of reverse-phase silica gel (C-18) was equilibrated in a sintered glass column with water: methanol (9:1 v/v). The impure product dissolved in 30 ml of water: methanol (9:1 v/v) was then applied to the equilibrated column and eluted with 150 ml of water: methanol (9:1 v/v), followed by an additional 150 ml of water: methanol (4:6 v/v). The purified product eluted in the latter solvent system, and the eluant was rotary-evaporated to a volume of approximately 5 ml and then lyophilized. Total yield of purified compound II was 161 mg and the purity was found to be >95% by TLC on silica gel plates using a 1:1:1 butanol:ethanol:0.880 ammonia solvent.

A total of 70 ml of Toyopearl AF-Carboxy-650M resin was then washed with 300 ml 1,4-dioxane to facilitate the removal of storage buffer. The resin was then equilibrated by shaking at ambient temperature in 60 ml 1,4-dioxane containing 4.03 gm of 1-(3-dimethylaminopropyl)-3-ethylcarbodiimide hydrochloride, 3.0 ml of diisopropylethylamine, and 2.41 gm of N-hydroxysuccinimide for 3 hours. The resin was next transferred to a chromatography column and washed with approximately 100 ml of 1,4-dioxane, followed by 300 ml of dimethyl sulfoxide (DMSO), and then divided (as a DMSO-based slurry) into a total of three 20 ml lots (*i*.*e*., 20 ml settled volume).

### (i) Resin Loading at 10 µMol/ml

To one of the 20 ml lots was added 200 µM (*i*.*e*., 137.2 mg) of Compound II dissolved in 20 ml of DMSO, and the mixture was shaken at ambient temperature for 3 hours. Following this incubation, a small sample of the supernatant was spotted on a silica gel TLC plate, dried under vacuum, chromatographed using a 1:1:1 butanol:ethanol:0.880 ammonia solvent system, and then visualized by dipping in 1% rescreinol:1% sulfuric acid mixture in isopropanol and heating to 400 °C. Results indicated that less-than 2% of the original concentration of trisaccharide could be detected, thus demonstrating that almost complete bonding to the resin had occurred. Ethanolamine (0.24 ml) was added to the original mixture and shaking was continued for an additional 2 hours at ambient temperature (*i*.*e*., 19 °C to 26 °C). The resin was then transferred to a chromatography column and washed with approximately 800 ml of 5 °C water. The column was stored with the resin equilibrated in water containing 0.02% azide.

### (ii) Resin Loading at 1.0 µMol/ml

The resin was essentially processed as above, except that 13.7 mg of Compound II was dissolved in 20 ml of DMSO, and the mixture was shaken at ambient temperature for 2 hours. The reaction was visualized as above. Results indicated that no trisaccharide could be detected, thus demonstrating that complete bonding to the resin had occurred.

### (iii) Resin Loading 0.1 µMol/ml

A similar procedure was followed to produce resin loaded at 0.1 µMol/ml, except that 1.37 mg of Compound II was dissolved in 20 ml of DMSO, and the mixture was shaken at ambient temperature for 2 hours. The reaction was visualized as above. Results indicated that no trisaccharide could be detected, thus demonstrating that complete bonding to the resin had occurred.

### EXAMPLE 3 Affinity Chromatography

One ml of the Group B trisaccharide-conjugated resin (*i*.*e*., Toyopearl AF-Carboxy-650M methacrylate resin) was packed into a 1x10 cm column and washed with a total of 20 column volumes of 150 mM Sodium Citrate, pH 7.4, to rinse away any preservatives in the resin. The washed, equilibrated resin was then loaded with 30 ml of plasma at a flow rate of 0.25 ml/min (residence time 4 minutes). All processing was performed at room temperature (20-25 °C).

During sample elution, 5 ml fractions were collected and fractions from approximately every fifth cycle were saved for subsequent analysis. Samples were taken from the starting material and maintained at room temperature until all fractions from a given chromatography run were collected. The samples were frozen and thawed simultaneously so as to correct for any possible loss of activity which may have occurred during collection and storage. Plasma samples were assayed for anti-B IgM immunoglobulin removal by the Immediate Spin (IS) agglutination test and for anti-B IgG immunoglobulin removal by the Indirect Coombs Test (ICT). Maintenance of the biological activity of coagulation factors V, VII, VIII, IX, X, XI, XII and XIII was assayed by the one-stage partial thromboplastin time. Additionally, the absorption at 280 nm was monitored for each chromatography run and the elution absorption profile recorded.

A similar analysis was performed using a larger (5 ml) column volume to reconfirm recovery of coagulation factors and to analyze eluted constituents. The eluted fractions were dialyzed a total of 5-times against 100-volumes of 25 mM Tris-HCl, pH 7.0, and electrophoresed on a reducing 8% SDS-PAGE gels. Starting S-D Plasma, as well as the (unadsorbed plasma) pass through fraction underwent gel electrophoretic analysis. In subsequent large-scale production, plasma pool formation (*i*.*e*., pre-pooling) and pooling of each lot was performed in a blood group type-specific manner.

### EXAMPLE 4 Automated Chromatography

Pooled Solvent-Detergent (SD) Plasma and regeneration buffers were connected to two 4-inlet manifolds whose open/close valves are electrically controlled by a programmable timing control unit (Kearsarge Model 831, 8-channel Universal Timer; Reston, VA). The solutions were pumped onto each of the two 1x10 cm chromatography columns containing 1 ml resin by a peristaltic pump (Pharmacia P-3 pump; Upsala, Sweden), calibrated for a 0.25 ml/mm flow rate. The outlet of each column was oriented so as to pass through an in-line 280 nm detector (LKB 2138 UVICORD 5; Brommer, Sweden) and recorder (Pharmacia Model 483 Strip Chart Recorder) and finally to a fraction collector (LKB 2112 REDIRAC collector). Five (5) ml fractions were collected. The timer controller was programmed to both load and regenerate the chromatography columns continuously for 34 cycles/week for up to 4 weeks. Chromatography was stopped on Friday and restarted on Monday. During this down-time, the resins were stored in 20% ethanol at 4 °C. Following this storage (*i*.*e*., at the beginning of each week), the resins were resuspended, fully regenerated and equilibrated, and the chromatography resumed.

### EXAMPLE 5 Regeneration of the Chromatography Resin

A 1 mL column containing the polymethacrylate resin-B antigen combination was used and regenerated for over 100 cycles without the loss of function. After loading of the plasma sample, a one column-volume (CV) wash was performed to recover plasma from the resin and tubing prior to the beginning of the regeneration procedure. The use of three CV was used for buffer exchange of the resins in the column. This volume is consistent with the regeneration procedures which have been used in the art for SD-plasma and for regeneration of fibrinogen affinity ToyoPearl resin. A total of six CVs of 150 mM Citrate, pH 7.4 equilibration buffer was used for resin pH adjustment in order to ensure pH neutralization and removal of any potential contaminants in a production environment prior to reloading the column. The basic protocol for use and regeneration of a 1 ml polymethacrylate-based affinity column is outlined in Table 1.

**Table 1**

| **STEP** | **VOLUME** | **BUFFER** |
|---|---|---|
| Load | 30 ml | Blood Composition |
| Wash | 1 ml | 150 mM Citrate, pH 7.4 |
| Regeneration 1 | 3 ml | 3M Sodium thiosulfate (Na₂S₂O₃) |
| Regeneration 2 | 3 ml | 6M Urea |
| Wash | 0.5 ml | 30%EtOH |
| Regeneration 3 | 3 ml | 70% EtOH/ 2% acetic acid |
| Equilibration | 6 ml | 150 mM Citrate, pH 7.4 |

The results of the regeneration studies demonstrated that: (*i*) there are no detectable changes in the amount of the carbohydrate-based ligand present on the resin following a total of 103 recycles; and (*ii*) the analysis of the eluate from the resin, consisting mainly of protein, showed no significant change between cycles 1 and 103.

Coagulation factors V, VII, X, and XI were recovered in high yields with the for the Group B trisaccharide-Toyopearl AF-Carboxy-650M conjugated resin throughout more than 200 cycles. As shown in Table 2, the recovery of coagulation factors was consistently close to 100%.

**Table 2:**

| **B-Resin Recycling Study** | | | | | | | | |
|---|---|---|---|---|---|---|---|---|
| Sample | FV u/ml | % REC | FVII u/ml | % REC | FX u/ml | % REC | FXI u/ml | % REC |
| Pre recyc. Start | 1.1 | 100% | 1.14 | 100% | 1.36 | 100% | 1.05 | 100% |
| Pre recyc. Pass | 1.07 | 97% | 1.06 | 93% | 1.24 | 91% | 1.03 | 98% |
| Thru | | | | | | | | |
| Pt 20 recyc. | 1.04 | 95% | 1.25 | 110% | 1.39 | 102% | 0.98 | 93% |
| Pt 40 recyc. | 1.02 | 93% | 1.22 | 107% | 1.47 | 108% | 0.98 | 93% |
| Pt 72 recyc. | 1.04 | 95% | 1.36 | 119% | 1.33 | 98% | 0.99 | 94% |
| Pt 95 recyc. | 0.947 | 86% | 1.29 | 113% | 1.39 | 102% | 0.99 | 94% |
| Pt 147 recyc. | 1.07 | 97% | 1.6 | 140% | 1.39 | 102% | 0.94 | 90% |
| Pt 202 recyc. | 1.1 | 100% | 1.56 | 137% | 1.33 | 98% | 1.02 | 97% |
| Plasma = SD-Plasma FE | | | | | | | | |
| 70918 | | | | | | | | |
| 0.05% Peracetic acid/ 20% ETOH/ 0.01M Na Acetate, pH 5.0 | | | | | | | | |
| B-Trisaccharide resin | | | | | | | | |

### EXAMPLE 6 Determination of Isoagglutinin Depletion

Following collection, samples from an affinity chromatography-mediated B-isoagglutinin depletion using B trisaccharide-Toyopearl AF-Carboxy-650M conjugated resin were frozen at -80 °C until assayed. Antibody titer determinations were performed according to procedures set forth in the AABB Technical Manual. For the Immediate Spin Agglutination Test for anti-B IgM immunoglobulin detection, two drops of the sample plasma were placed into a labeled 12x75 glass test tubes and 2-fold serially diluted in normal saline over an 8-dilution series. One drop of 3 % reagent red blood cells (CONFIRMCELLS Group B; BGA, West Chester, PA) was added to each tube, mixed gently, and centrifuged at 1000 X g for 1 minute. The collected "button" of erythrocytes was then gently shaken off the wall of the tube and examine for signs of agglutination. All reactions are read macroscopically and scored from a value of 4 to 12 based upon the scoring system of Marsh. See Marsh, *et. al., Transfusion* **12**: 352-353 (1972). Conversely, any score below a value of 4 can only be quantified microscopically, and is not deemed to be clinically relevant for transfusional purposes. The first tube in the dilution series where no agglutination appears is scored as a 0, and recorded as the end-point titer.

In the determinations using the Indirect Coombs Test (ICT) for Anti-B IgG immunoglobulin detection, the tubes are further incubated at 37°C for 30 minutes, followed by a centrifugation step to facilitate removal of the supernatant. The collected erythrocytes are then washed and re-centrifuged a total of 3-times with I ml of normal saline, and the supernatant carefully and completely decanted following the final wash. Two drops of poly-specific Anti-IgG-C3d Anti-Human Globulin was added to each tube, gently mixed, and centrifuged. Each tube is scored in an identical manner as described above for the Immediate Spin Agglutination Test for anti-B IgM immunoglobulin detection.

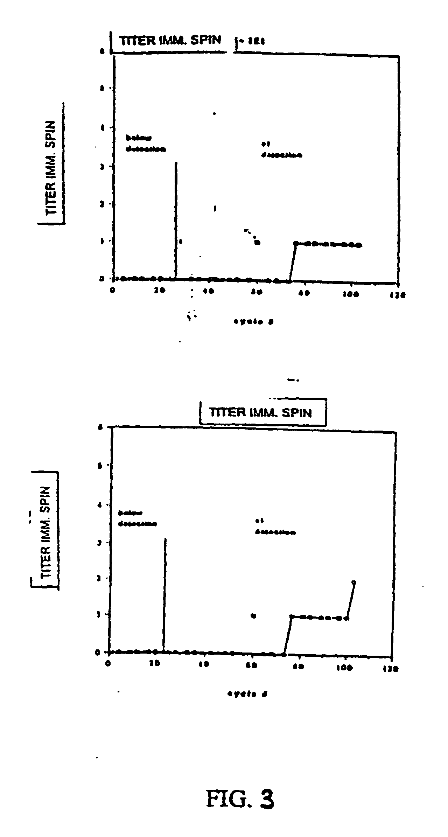

FIG. 3 illustrates the titers of anti-B IgM and anti-B IgG immunoglobulins present in the pooled-plasma pass through fractions of different cycles (one cycle is equivalent to the use and regeneration of the column). The capacity for anti-B IgM retention with the Group B trisaccharide-Toyopearl AF-Carboxy-650M conjugated resin was excellent, as antibody could not be detected in undiluted plasma sample for the first 25-30 recyclings. In subsequent cycles, it was found that there was a 4-fold titer dilution removal of anti-B IgM immunoglobulin for approximately 45 recyclings, followed by an 8-fold dilution removal throughout the 103 total recyclings. The absorption was demonstrated to be very similar for anti-B IgG immunoglobulin removal, although the overall retention was reduced. One possible explanation of this result may be due to the fact that IgM is a pentameric immunoglobulin, thus due to large size and high number of binding sites, it may bind to many more antigenic sites than the IgG immunoglobulin. Specifically, throughout a total of 100 cycles, 1 ml of the Group B trisaccharide-Toyopearl AF-Carboxy-650M conjugated resin was found to be capable of removing 3-fold titer dilution of anti-B IgM and IgG in three liters of plasma, with residual IgM and IgG titers of 2¹ and 2¹, respectively.

An additional confirmatory study was performed in order to determine the titer of anti-B immunoglobulins remaining in blood group type A plasma following immunoselection with the Group B trisaccharide-Toyopearl AF-Carboxy-650M conjugated resin. Column elution fractions were taken after 9 regeneration cycles and 89 regeneration cycles of the resin, and the fractions were tested for anti-B IgM (immediate spin) and for anti-B IgG (by indirect Coombs testing). A two-step complement hemolysis test was performed along with adsorption and elution studies using a commercial acid elution kit. The following results (summarized in Table 3) were obtained: (*i*) no positive agglutination reactions were found after 9 cycles at immediate spin, with only a slight increase after 89 cycles at immediate spin; (*ii*) the addition of fresh complement showed no evidence of hemolysis with the same samples; and (*iii*) the elution study was negative for both samples. Thus, patients who are transfused across blood groups would not have the SD-Plasma-derived isoagglutinin implicated as a source of the reaction, because the antibody does not elute from the erythrocytes.

**Table 3:**

| **Results of Hemagglutination Tests of B-Resin Recycling Study** | | | | | | | | | | | | | | | | | | | | |
|---|---|---|---|---|---|---|---|---|---|---|---|---|---|---|---|---|---|---|---|---|
| | Cycle 9 fraction 4 | | | | | Cycle 9 fraction 6 | | | | | Cycle 89 fraction 4 | | | | | Cycle 89 fraction 4 | | | | |
| RBC standard | IS | RT | 4°C | 37°C | IAT | IS | RT | 4°C | 37°C | IAT | IS | RT | 4°C | 37°C | IAT | IS | RT | 4°C | 37°C | IAT |
| B Gamma #210 | 0 | 5 | 7 | 5 | 7 | 0 | 6 | 6 | 6 | 6 | 5 | 10 | 10 | 7 | 10 | 6 | 8 | 10 | 10 | 9 |
| B Gamma #224 | 0 | 6 | 7 | 5 | 6 | 0 | 6 | 7 | 6 | 5 | 5 | 8 | 10 | 8 | 10 | 6 | 9 | 11 | 9 | 8 |
| OP #876 | 0 | 0 | 0 | 0 | 0 | 0 | 0 | 5 | 5 | 0 | 0 | 0 | 7 | 0 | 0 | 0 | 0 | 5 | 0 | 0 |
| OP #08 | 0 | 0 | 0 | 0 | 0 | 0 | 0 | 5 | 0 | 0 | 0 | 0 | 6 | 0 | 0 | 0 | 0 | 5 | 0 | 0 |
| Results are given in hemagglutination scores of 1 - 12, with 12 as the highest agglutination | | | | | | | | | | | | | | | | | | | | |
| IS = Immediate Spin | | | | | | | | | | | | | | | | | | | | |
| RT = Room Temperature | | | | | | | | | | | | | | | | | | | | |
| IAT = Indirect Antiglobulin Test | | | | | | | | | | | | | | | | | | | | |
| Cycle 9 and cycle 89 are from the respective cycles of the recycling study | | | | | | | | | | | | | | | | | | | | |
| Fraction 4 and 6 are fractions from the eluted plasma pool of a particular cycle. | | | | | | | | | | | | | | | | | | | | |

### EXAMPLE 7 Coagulation Factor Assays

Following collection, samples for coagulation factor determination were frozen at -80 °C until assayed. Each sample was assayed in duplicate and the results averaged. Coagulation factor activities for factors VIII and XI were determined by the one-stage activated partial thromboplastin time clotting assay (APTT) on a Sysmex CA-5000 Automated Coagulation Analyzer (TOA Medical Electronics; Los Alamitos, CA). The APTT time measures the degree of correction of the clotting time of plasma deficient factor (George King Biomedical; Overland Park, KS) in the presence of APTT reagent (Organon-Teknika; Durham, NC). Factor V and VII activities were assayed similarly, except thromboplastin with calcium (Sigma Diagnostics; St. Louis, MO) was utilized in place of the APTT reagent. Biological activity in units/ml was then determined by comparison to a standard curve using a calibrated standard plasma (Instrumentation Laboratories; Lexington, MA).

Additionally, separate samples were also saved and stored for longer-term stability studies at -30 °C. These samples were to be assayed for retention of coagulation factor activity after 6 months.

**Table 4:**

| **Coagulation Factor Recovery and Isoagglutinin Levels in Universal.Plasma** | | | | | | | | |
|---|---|---|---|---|---|---|---|---|
| Lot # | Anti-A | Anti-B | FV | FVII | FX | FXI | FXIII | Protein |
| UNV060199 | - | Undetectable | 0.9 | 1.1 | 1.1 | 0.9 | 0.9 | 6.10 |
| FW90601 | - | 1:16 | 0.9 | 1.0 | 1.1 | 0.9 | 1.0 | 6.20 |
| UNV060899 | - | 1.2 | 0.9 | 1.3 | 1.1 | 0.9 | 1.0 | 6.30 |
| FW90608 | - | 1:64 | 0.8 | 1.0 | 1.1 | 1.0 | 1.0 | 6.30 |
| UNV061099 | 1:1 | Undetectable | 0.9 | 1.3 | 1.1 | 0.9 | 0.9 | 6.20 |
| FZ90610 | 1:32 | 1:64 | 0.8 | 1.1 | 1.0 | 0.9 | 0.9 | 6.1 |
| UNV061599 | - | Undetectable | 0.8 | 1.2 | 1.1 | 0.9 | 1.0 | 5.90 |
| FW90515 | - | 1:64 | 0.9 | 1.1 | 1.1 | 1.0 | 1.0 | 6.10 |
| UNV061799 | 1:2 | 1:1 | 0.9 | 1.6 | 1.2 | 0.9 | 0.8 | 6.10 |
| FZ90617 | 1:64 | 1:64 | 0.8 | 1.0 | 1.0 | 0.9 | 1.0 | 6.10 |
| UNV052399 | Undetectable | Undetectable | 0.8 | 1.2 | 1.1 | 0.9 | 0.8 | 6.2 |
| FZ90623 | 1:64 | 1:64 | 0.9 | 1.1 | 1.1 | 1.0 | 0.9 | 6.2 |
| UNV063099 | Undetectable | Undetectable | 0.8 | 1.6 | 1.2 | 0.8 | 0.9 | 6.2 |
| FZ90630 | | | 0.8 | 1.4 | 1.1 | 0.9 | 1.0 | 6.1 |
| UNV070899 | Undetectable | Undetectable | 0.8 | 1.2 | 1.1 | 0.8 | 0.9 | 6 |
| FZ90708 | | | 0.9 | 1.1 | 1.1 | 0.9 | 1.0 | 5.6 |
| UNV071499 | | | 0.7 | 1.4 | 1.1 | 0.8 | 0.9 | 6.1 |
| FZ90714 | | | 0.8 | 1.0 | 1.1 | 0.9 | 1.0 | 6.3 |

| UNIVERSAL PLASMA | | | | | | | | |
|---|---|---|---|---|---|---|---|---|
| Mean | | | 0.83 | 1.32 | 1.12 | 0.87 | 0.90 | 6.12 |
| SD | | | 0.07 | 0.18 | 0.04 | 0.05 | 0.07 | 0.12 |
| Range | Undect - 1:2 | Undect-1:2 | 0.7 - 0.9 | 1.1-1.6 | 1.1 - 1.2 | 0.8-0.9 | 0.8 - 1.1 | 5.9-6.3 |

| PARENT PLASMA | | | | | | | | |
|---|---|---|---|---|---|---|---|---|
| Mean | | | 0.84 | 1.09 | 1.08 | 0.93 | 0.98 | 6.11 |
| SD | | | 0.05 | 0.13 | 0.04 | 0.05 | 0.0 | 0.2 |
| Range | 1:32-1 | 1:16-1:64 | 0.8-0.9 | 1.0-1.4 | 1.0-1.1 | 0.9-1.0 | 0.9-1.0 | 5.6-6.3 |
| UNV is the abbreviation for Universal and FW is A-Plasma and FZ is O-Plasma. | | | | | | | | |

### EXAMPLE 8 Plasma Protein Assays

*SD plasma* that has been prepared according to the methods of the invention were tested for loss of proteins other than the targeted isoagglutinins. As mentioned above, a common problem with many methods for isoagglutinin removal is that they have a high rate of loss of indigenous protein. These proteins include antibodies to viruses such as hepatitis A virus (HAV), blood clotting factors, and other factors (*e*.*g*., kallikrein and von Willebrand factor [vWF] metalloproteinase. A comparison of protein percentage present in control (parent) and universal (unadsorbed, isoagglutinin depleted) plasma is shown in Tables 5-7.

**Table 5:**

| **Determination of anti-B19 antibody Level** | | |
|---|---|---|
| **Cycle** | **Sample** | **Anti-B19 antibody (mIU/ml)** |
| 17 | Control | 9.19 |
| 17 | Unabsorbed plasma fraction #4 | 8.17 |
| 17 | Unabsorbed plasma fraction #6 | 8.35 |
| 49 | Control | 10 |
| 49 | Unabsorbed plasma fraction #4 | 9.17 |
| 49 | Unabsorbed plasma fraction #6 | 10.15 |
| 97 | Control | 10.86 |
| 97 | Unabsorbed plasma fraction #4 | 11.61 |
| 97 | Unabsorbed plasma fraction #6 | 9.25 |
| Control = Start SD-Plasma FE 70918 | | |
| Cycles 17, 49, and 89 are from the respective cycles of the recycling study | | |
| Fraction 4 and 6 are fractions from the unabsorbed plasma pool of a particular cycle. mIU/ml = milli International Units per ml sample | | |

**Table 6:**

| **Determination of HAV Antibody Levels** | | |
|---|---|---|
| **Cycle** | **Sample** | **Anti-HAV antibody (IU/ml)** |
| 17 | Control | 2.4 |
| 17 | Unabsorbed plasma fraction #4 | 2.32 |
| 17 | Unabsorbed plasma fraction #6 | 2.29 |
| 49 | Control | 2.37 |
| 49 | Unabsorbed plasma fraction #4 | 2.32 |
| 49 | Unabsorbed plasma fraction #6 | 2.36 |
| 97 | Control | 2.16 |
| 97 | Unabsorbed plasma fraction #4 | 2.11 |
| 97 | Unabsorbed plasma fraction #6 | 2.05 |
| Control = Start SD-Plasma FE 70918 | | |
| Cycles 17, 49, and 89 are from the respective cycles of the recycling study | | |
| Fraction 4 and 6 are fractions from the unabsorbed plasma pool of a particular cycle. IU/ml = International Units per ml sample | | |

**Table 7:**

| **Pre-Kallikrein and Kallikrein Values in Pilot Scale Lots** | | | | |
|---|---|---|---|---|
| | **Pre-Kallikrein Activator (mU/ml)** | | **Kallikrein (mU/ml)** | |
| **Lot No.** | **Universal** | **Parent** | **Universal** | **Parent** |
| UNV060199 | 33 | 103 | 6.96 | 4.73 |
| UNV060399 | 83 | 90 | 8.21 | 4.73 |
| UNV061099 | 47 | 52 | 12.67 | 7.52 |
| UNV061599 | 95 | 117 | 8.49 | 5.85 |
| UNV061799 | 63 | 91 | 14.06 | 6.54 |
| UNv062399 | 85 | 85 | 11.14 | 5.57 |
| UNv063099 | 60 | 73 | | |
| **Average:** | 66.6 | 87.3 | 10.3 | 5.8 |
| | 22.3 | 20.8 | 2.8 | 10.8 |

Additional experiments were performed in order to determine the activity of von Willebrand factor (vWf) cleaving metalloproteinase in pooled *SD plasma* as well as in isoagglutinin-depleted *SD plasma*. Removal of anti-A or anti-B isoantibodies using anti-A or anti-B trisaccharides linked to synthetic resins does not detectably reduce vWf-cleaving metalloproteinase activity in treated *SD plasma*.

### EXAMPLE 9 Stability Over Time of Isoagglutinin-Depleted Plasma

The stability of plasma over time was examined by measuring various plasma parameters, alpha 1 globulin, alpha 2 globulin, beta globulin, gamma globulin, Factor V, Factor VII, Factor X, Factor XI, Factor XIII, and fibrinogen. Levels of hepatitis A virus (HAV) and parvovirus including levels of plasma proteins, infectious agents, and pH. Proteins examined included albumin19 nucleic acids were also measured, as was pH.

Plasma samples were stored at -18°C. Nine separate lots of isoagglutinin-depleted plasma were examined. For each lot, the indicated parameter was measured at 0, 3, 6, 9, and 12 months after preparation of the serum. No significant difference was found in the levels of the proteins or nucleic acids measured in the time points examined.; These results indicate that the isoagglutinin-depleted plasma was stable for at least 12 months.

### EXAMPLE 10 Removal of Anti-A Isoagglutinins and Anti-B Isoagglutinins from Solvent Detergent Treated type O Human Plasma

An A-saccharide resin was used to remove solvent detergent treated pooled human plasma using production scale operating parameters. The depletion of A and B isoagglutinins from Type O SD-Plasma appears to be independent of the order of the resins, *i*.*e*., removal of A followed by removal of B is equivalent to B followed by A. In this example, plasma that had been first depleted of anti-B isoagglutinins was used as the starting material for validating the removal of anti-A isoagglutinins.

B-trisaccharide resin was used at a ligand density of 1.0 µmol B-trisaccharide/ml Toyopearl (TosoHaas) resin to deplete type O SD-Plasma of B-isoagglutinins. A-trisaccharide resin at a ligand density of 1.2 µmol A-trisaccharide/ml resin was then used to further deplete the SD-Plasma of A-isoagglutinins. Both resins are manufactured by ProMetic BioSciences (Isle of Man, United Kingdom). The operating parameters were chosen to reflect production scale conditions. Bed height was 1.27 - 20 cm with a target bed height of 12.7 cm. Other parameters included a linear flow of 127-380 cm/hr, with a target linear flow of 190 cm/hr, a load ratio of 20:1 - 40:1, plasma volumes to resin volumes with a target load ratio of 30:1 plasma volumes to resin volumes, pH 7.0 - 8.0 with a target of 7.4, and temperature 20° - 25 °C with a target temperature of 23 °C.

A target plasma load to resin ratio of 30:1 was selected as being representative of likely production operating conditions. Intermittently, 20:1 and 40:1 ratios were evaluated to test the extremes of the load ranges.

All chemicals were USP grade unless otherwise stated. Trisodium citrate, (cat. no. SX0444-1 and sodium thiosulfate (cat. no. SXO813-1) were obtained from EM Science, (Gibbstown, NJ). Glacial acetic acid (cat. no. 9522-021), urea (cat. no. 8642), and sodium chloride (cat. no. 7540) were obtained from Mallinckrodt (Paris, Kentucky). Redistilled 95% EtOH was obtained from VITEX's Melville Manufacturing site. Reagent red blood cells (Immmucor/Gamma Biologicals, Inc. Houston, TX) and polyspecific IgG,-C3d anti-human Globulin were obtained from Immucor Inc. (Norcross, GA).

Pooled type O solvent-detergent treated plasma was stored in a -30°C freezer until ready for use. B-trisaccharide resin chromatography was performed using a 2.5 x 20 cm column containing 50 ml B-trisaccharide resin at a ligand density of 1.0 µmol B- trisaccharide/ml resin was prepared and equilibrated with 10 column volumes (CV) of 0.15M NaCl. Type O SD-Plasma was loaded onto the 50 ml resin column at a flow rate of 9.1 ml/min representing a contact time of 5.5 min to deplete the plasma of anti-B isoagglutinins prior to loading onto the A-trisaccharide resin. The bed height of 10.2 cm and linear flow velocity of 111 cm/hr will approximate the bed height (12.7 cm) and linear flow (190 cm/hr) of the 1 liter resin column used in production scale-up runs. All chromatography was performed at ambient temperature.

Seven units (1,400 ml) of type O SD-Plasma were thawed, and the thawed plasma was adjusted to pH 7.4 with O'IN HCL and loaded at a ratio of 30:1, plasma to resin, onto the B-trisaccharide resin. The plasma was chromatographed at a contact time of 5.5 mm at 20° - 25°C (RT) to remove anti-B isoagglutinins.

The B-isoagglutinin-depleted type O SD-Plasma was used as the start plasma for the A-trisaccharide resin for the removal of A-isoagglutinins. The B-trisaccharide resin was then fully regenerated, stored overnight in 95% ethanol at room temperature (RT) and re-equilibrated the following day in 10 CV 0.15M NaCl prior to re-usage.

A-Trisaccharide resin chromatography was performed using ten (10) ml of the A-trisaccharide resin packed under gravity and allowed to settle into the bottom of a 1 x 20 cm Bio Rad borosilicate glass column. This mimicked the bed height of 12.7 cm and linear flow velocity of 191 cm/hr to be used in production scale and pilot scale runs. The resin was washed with ten (10) column volumes of 0.15 M NaCl prior to use.

Three hundred (300) ml of B-isoagglutinin depleted type O pooled SD-Plasma was loaded per cycle onto the 10 ml A-trisaccharide resin column at a flow rate of 9.1 ml/min (111 cm/hr), which represents a residence time of 5.5 mm. The sequence for chromatography steps is given below. After cycles 12, 46 and 76, the resin was challenged first with 20 CV plasma, regenerated and followed by 40 CV of plasma to test the extremes of the protein load range. After a week of cycling (*e.g*., four cycles per day, sixteen cycles per week), the column was washed and equilibrated with 95% alcohol and then stored at RT. At the beginning of the following week the column was washed and equilibrated with 10 CV of 0.15M NaCl and recycling resumed. The recycling continued throughout 106 recycles.

**Table 8:**

| **Recycling protocol for A-Resin** | | |
|---|---|---|
| **STEP** | **CV** | **SOLUTION** |
| Load | 30 | SD-Plasma |
| Wash | 1 | 0.15M Sodium Chloride |
| Regeneration 1 | 3 | 3M Sodium Thiosulfate, pH 7.4 |
| Regeneration 2 | 3 | 6M Urea |
| Wash | 0.5 | 16.9% Ethanol |
| Regeneration 3 | 3 | 70% Ethanol//2% Acetic Acid (w/w) |
| Equilibration | 6 | 0.15M Tri-sodium Citrate, pH 7.4 |
| Equilibration 2 | 10 | 0.15M Sodium Chloride |

All processing was performed at room temperature (RT).

Fifty (50) ml fractions were collected and fractions from approximately every fourth cycle were saved for analysis. Samples were taken from the starting material and maintained at room temperature until all fractions from that chromatography run were collected. The samples were frozen at -30 °C. All samples were collected and thawed simultaneously to correct for any possible loss activity during collection, storage and freeze-thawing. Prior to analysis, the samples were thawed simultaneously in a 37 °C H₂O bath.

Plasma samples were assayed for anti-A and anti-B IgM removal by the Immediate Spin Test (IS) and for anti-A and anti-B IgG removal by the Indirect Coombs Test (ICT). Coagulation factors V, VII, X, and XI activity was assayed by the one-stage partial thromboplastin time. The absorption at 280 nm was monitored for each chromatography run and the profile recorded.

For automated chromatography, B-isoagglutinin depleted type O pooled SD-Plasma and regeneration buffers were connected to two 4-inlet manifolds whose open/close valves are electrically controlled by a programmable timing control unit (Kearsarge model 831 8-channel universal timer, Reston, VA). The solutions were pumped onto the 1 x 20 cm column containing 10 ml resin by a peristaltic pump (Pharmacia P-3 pump, Upsala, Sweden) calibrated to flow at 1.8 ml/mm (137 cm/hr). The column passed through an in-line UV absorbance A280 detector (LKB 2138 UVICORD S, Brommer, Sweden) and recorder (Pharmacia strip chart recorder, model 483) and finally to a time collector (LKB 2112 REDIRAC collector). Fifty (50) ml fractions were collected. The time controller was programmed to load and regenerate the columns continuously for 16 cycles/week for 8 weeks. Chromatography was stopped on Thursday and resumed on Monday. During this time the resins were stored in 95% alcohol at RT for the weekend.

Samples for isoagglutinin removal assay were frozen at -30°C until assayed. Antibody titer determinations were run according to the standard procedures outlined in the AABB Technical Manual (p 555-564, 10th Ed., 1990). For the Immediate Spin Test (IS) for anti-A and anti-B 1gM detection, two (2) drops of sample plasma were placed into labeled 12 x 75 glass test tubes and 2-fold serially diluted in normal saline over 8 dilution series. One (1) drop of 3 % reagent red blood cells (Immucor Group B cells/Gamma Biol, Houston, TX) was added to each tube. The tubes were mixed and then centrifuged at 1000 x g for 1 mm. The red cell button was then gently shaken off the wall of each tube and examined for agglutination. All reactions were read macroscopically and scored from a value of 4 to 12 based on the scoring system of Marsh. Marsh *et. al*., Transfusion, 12: 352-353, 1972. Any score below 4 can only be quantified microscopically and is not clinically relevant for transfusion purposes. The first tube in the dilution series where no agglutination appeared was scored as a 0 and recorded as the end point titer. For the Indirect Coomb's Test (ICT) for anti-A and anti-B IgG detection, the tubes were further incubated at 37°C for 30 min and then centrifuged to remove the supernatant. The cells were then washed and centrifuged 3 times with 1 ml normal saline and the supernatant carefully decanted completely after the last wash. Two (2) drops of polyspecific Anti-IgG,-C3d Anti-Human Globulin (Imnucor, Norcross, GA.) was added to each tube. The tubes were mixed, centrifuged, and finally scored as above.

Samples for coagulation factor determination were frozen at -30°C until ready for assay. Each sample was assayed in duplicate and the results averaged. Coagulation factor activity for factor X and XI was determined by the one-stage activated partial thromboplastin time clotting assay (APTT) on a Sysmex CA-5000 Automated Coagulation Analyzer (TOA Medical Electronics, Los Alamitos, Ca). The APTT time measures the degree of correction of the clotting time of plasma deficient factor (instrumentation Laboratories, Lexington, MA) in the presence of APTT reagent (Instrumentation Laboratories). Factor V and VII activity was assayed similarly, except PT-Fibrinogen with calcium (Instrumentation Laboratories) replaced the APTT reagent. Biological activity in units/ml was determined by comparison to a standard curve using a calibrated standard plasma (Instrumentation Laboratories).

The OD profiles at 280 nm of the flow through of the resin for early cycles, mid cycles. and late cycles were comparable. All the load profiles, including the low and high plasma load ratios, appeared comparable. Most regeneration profiles appeared identical, except for post cycle 1 and cycle 3 re-equilibration baseline, which shifted upward, and for the cycles near the end of the recycling study where the OD tracing of the regeneration procedure appear not to drop as precipitously to the baseline as in the early cycles. The reason for the discrepancies is not known. This material is not collected during manufacture and will not have any product impact.

Recovery of coagulation factors V, VII, X, and XI were satisfactory for the resin throughout the 102 cycles (108 cycles when the extreme protein load cycles are included). Most pre-column and post-column values varied within 10 %. The mean and standard deviation were 1.0 u/ml and 0.0701 for pre-column and 1.0 u/ml and 0.0820 for post B-column FV and 0.9 u/ml and 0.0522 for post A-column FV; 1.0 u/ml and 0.0505 for pre-column and 1.0 u/ml and 0.0505 for post B-column FVII and 1.0 u/ml and 0.0539 for post A-column FVII; 1.0 u/ml and 0.0505 for pre-column and 1.0 u/ml and 0.0505 for post B-column FX and 1.0 u/ml and 0.0000 for post A-column FX; and finally 0.9 u/ml and 0.0459 for pre-column and 0.9 u/ml and 0.0688 for post B-column FXI and 0.8 and 0.0820 for post A-column FXI.

The titers of both anti-A & anti-B IgM (IS) and IgG (ICT) present in the pooled plasma flow through fractions of different cycles at a load ratio of 30:1, plasma to resin were examined. There was a 6-7 doubling fold dilution or 98 % - 99 % reduction of both anti-B IgM (18) and anti-B IgG (ICT) throughout the 102 cycles with a post column flow through plasma titer 1 and 2 to 4 respectively. For anti-A removal, there was a 5 doubling fold dilution or 97 % reduction of anti-A IgM (IS) with a post column flow through plasma titer of 1 throughout cycle 48 and a titer of 2 throughout cycle 102; for anti-A IgG (ICT) removal, there is a 6 doubling fold dilution reduction or 98 % throughout cycle 19 with a post column flow through plasma titer of 4 and a 5 doubling fold dilution reduction or 97 % throughout cycle 102 with a post column flow through plasma titer of 8.

At a load ratio 20:1 plasma to resin, the titers for anti-B IgM (IS) and IgG (ICT) were 0 and 2 respectively for cycle 12, 1 and 4 for cycle 46, and 0 and 2 for cycle 76. For anti-A, the IgM (IS) and IgG (ICT) titers were 1 and 4 respectively after cycle 12, 1 and 8 after cycle 46, and 1 and 8 after cycle 76.

At a load ratio of 40:1, plasma to resin, the titers for anti-B IgM (IS) and IgG (ICT) were 0 & 2 respectively for cycle 12 and 1 and 4 for cycle 46, 1 and 2 for cycle 76. For anti-A, the IgM (IS) and IgG (ICT) titers were 0 and 4, respectively, after cycle 12, 2 and 16 after cycle 46, and 2 and 8 after cycle 76.

This example demonstrates the efficacy of the A-trisaccharide resin prepared by the actual manufacturing process for removal of A-isoagglutinins. Ten (10) ml of A-trisaccharide resin with a bed height of 12.7 cm and linear flow 191 cm/hr was chosen to mimic full scale production including the one (1) liter resin columns used in pilot lot studies for the production of clinical grade material. However, due to the build-up of hydrostatic backpressure experienced during the chromatography of the B-trisaccharide resin, the linear flow of the A-trisaccharide was reduced to a contact time of 5.5 mm at a linear flow of approximately 140 cm/hr.

During the recycling study, the resin flow rate decreased with succeeding recycling from approximately cycle 72. Resin clumps were present on the top of the resin bed. This may be due to incomplete column regeneration caused by failure of electronic valves, which are responsible for the switching of different wash buffers. Because of this, the resin was resuspended and repacked a number of times throughout several cycles prior to replacement of the valves. After cycle 94, examination of the resin under low power magnification revealed an increased percentage of cleaved beads (-20 %) over that of virgin resin (~1-5 %). Magnification of the resin supernatant revealed undistinguishable inert microparticulate matter. It is probable that this microparitculate matter along with denser packing of the cleaved beads contributed to the decrease in flow raw of the resin. Subsequently, the column was repacked and fully regenerated routinely prior to every daily run of 4 cycles. The flow rate of the first cycle of each daily run approximated the designated flow rate (1.65 ml/min vs. 118 ml/min) but progressively decreased throughout the 4 cycle period. Of significance is that the overall performance of the resin was not affected as indicated by the results of the isoagglutinins removal and coagulation factors recoveries throughout the study.

Both B-trisaccharide resin and A-trisaccharide resin efficiently removed anti-B and anti-A isoagglutinins throughout 102 cycles to a final titer of ≤ 2 of each for the immediate spin (IS) and ≤ 8 for the indirect Coombs test (ICT) from type O plasma with starting titers of 64 for IS and 256 for ICT for anti-A antibodies and 64 (IS) and 128-256 (ICT) for anti-B antibodies. Coagulation factors V, VII, X, and XI recoveries remained satisfactory throughout the cycles and the chromatographic profiles were consistent throughout 102 cycles. Both A-resin and B-resin functional integrity remained intact throughout the study and is predictive of full production scale manufacture of anti-A and anti-B isoagglutinins depleted type O universal plasma.

### EQUIVALENTS

From the foregoing detailed description of the specific embodiments of the present invention, it should be readily apparent that a unique method for the production of blood-derived compositions, which have undergone depletion of blood group-specific isoagglutinins utilizing affinity chromatography, have been described. The particular blood-derived component which is selected for isoagglutinin-depletion is believed to be a matter of routine for a person of ordinary skill in the art with knowledge of the embodiments described herein.

## Claims

1. A method of regenerating a resin for removing an isoagglutinin from a blood-derived composition, the method comprising:
(a) providing a resin linked to antigen-isoagglutinin complex; and
(b) washing the resin with a regenerating buffer under conditions sufficient to selectively remove the isoagglutinin from the antigen-isoagglutinin complex, thereby regenerating the resin.

2. The method of claim 1, further comprising:
(c) contacting the regenerated resin with a blood-derived composition comprising said isoagglutinin under conditions sufficient to form a second antigen-isoagglutinin complex; and
(d) separating the blood-derived composition from the second antigen-isoagglutinin complex, thereby separating the blood-derived composition from the isoagglutinin.

3. The method of claim 2, wherein (b)-(d) are repeated at least twice.

4. The method of claim 2, wherein (b)-(d) are repeated at least 5 times.

5. The method of claim 2, wherein (b)-(d) are repeated at least 20 times.

6. The method of claim 2, wherein (b)-(d) are repeated at least 50 times.

7. The method of claim 1, wherein the regeneration buffer comprises sodium thiosulfate.

8. The method of claim 1, wherein the regeneration buffer comprises peracetic acid.

9. The method of claim 2, wherein the blood-derived composition is selected from the group consisting of whole blood, plasma, platelet preparations, fresh frozen plasma, solvent-detergent plasma, IVIG, IgM preparations, purified coagulation factor concentrates, and fibrinogen concentrate.

10. The method of claim 2, wherein the separating step removes blood composition proteins from the second antigen- isoagglutinin complex.

11. The method of claim 10, wherein the blood-based proteins include at least one of the blood coagulation factors: factor V, factor VII, factor VIII, factor IX, factor X, factor XI, factor XII and factor XIII; antibodies other than A- and B- isoagglutinins; indigenous proteins and enzymes; von Willebrand factor metalloproteinase, and kallikrein.

12. The method of claim 1, wherein the antigen comprises one or more trisaccharides.

13. A method for selectively removing an isoagglutinin from a blood-derived composition, the method comprising:
(a) providing a resin linked to an antigen specific for the isoagglutinin;
(b) washing the resin with a regeneration buffer to form a regenerated resin;
(c) contacting the washed resin with the blood-derived composition under conditions allowing for formation of an isoagglutinin-antigen complex on the resin;
(d) separating the blood-derived composition from the isoagglutinin-antigen complex; and
(e) repeating steps (b)-(d), thereby selectively removing the isoagglutinin from the blood-derived composition.

14. The method of claim 13, wherein steps (b) through (d) are repeated at least five times.

15. The method of claim 13, wherein steps (b) through (d) are repeated at least ten times.

16. The method of claim 13, wherein steps (b) through (d) are repeated at least 25 times.

17. The method of claim 13, wherein steps (b) through (d) are repeated at least 50 times.

18. The method of claim 13, wherein steps (b) through (d) are repeated at least 75 times.

19. The method of claim 13, wherein steps (b) through (d) are repeated at least 100 times.

20. The method of claim 13, wherein the regeneration buffer comprises sodium thiosulfate.

21. The method of claim 20, wherein the regeneration buffer comprises 3M sodium thiosulfate.

22. The method of claim 13, wherein the regeneration buffer comprises peracetic acid.

23. The method of claim 13, wherein the contacting is by column chromatography.

24. The method of claim 13, wherein the contacting is by batch absorption.

25. The method of claim 1 and claim 13, wherein the washing inactivates, renders non-infective, or removes, a virus or prion on the resin.

26. The method of claim 1 and claim 13, wherein the resin is a Toyopearl AF-Carboxy polymethacrylate resin.

27. A method for selectively removing an isoagglutinin from a blood-derived composition, the method comprising:
(a) providing a Toyopearl AF-Carboxy polymethacrylate resin linked to an antigen specific for the isoagglutinin;
(b) washing the resin with sodium thiosulfate to form a regenerated resin;
(c) contacting the washed resin with the blood-derived composition under conditions allowing for formation of an isoagglutinin-antigen complex on the resin;
(d) separating the blood-derived composition from the isoagglutinin-antigen complex; and
(e) repeating steps (b)-(d), thereby selectively removing the isoagglutinin from the blood-derived composition while retaining other blood proteins in the blood-derived composition.

28. The method of claim 27, wherein step (b) further comprises washing the resin with peracetic acid.

29. The method of claim 1, claim 13 and claim 27, wherein the isoagglutinin is anti-A isoagglutinin, anti-B isoagglutinin, or anti-A,B isoagglutinin.

30. A composition for binding an isoagglutinin, the composition comprising a polymethacrylate resin linked to an antigen specific for said isoagglutinin, wherein said antigen is linked to said resin by a carboxyl group on said resin.

31. The composition of claim 30, wherein said antigen is an oligosaccharide.

32. The composition of claim 31, wherein said antigen is a trisaccharide.

## Patentansprüche

1. Methode zum Regenerieren eines Harzes für das Entfernen eines Isoagglutinins von einer aus Blut gewonnenen Zusammensetzung, wobei das Methode Folgendes umfasst:
(a) Bereitstellen eines Harzes, das an einen Antigen-Isoagglutinin-Komplex angeknüpft ist; und
(b) Waschen des Harzes mit einem regenerierenden Puffermittel unter Bedingungen, die ausreichen, um das Isoagglutinin selektiv von dem Antigen-Isoagglutinin-Komplex unter Regenerierung des Harzes zu entfernen.

2. Methode nach Anspruch 1, des Weiteren umfassend:
(c) das Kontaktieren des regenerierten Harzes mit einer aus Blut gewonnenen Zusammensetzung, die das Isoagglutinin umfasst, unter Bedingungen, die ausreichen, um einen zweiten Antigen-Isoagglutinin-Komplex zu bilden; und
(d) Abtrennen der aus Blut gewonnenen Zusammensetzung von dem zweiten Antigen-Isoagglutinin-Komplex unter Abtrennung der aus Blut gewonnenen Zusammensetzung von dem Isoagglutinin.

3. Methode nach Anspruch 2, wobei (b) - (d) mindestens zweimal wiederholt werden.

4. Methode nach Anspruch 2, wobei (b) - (d) mindestens 5-mal wiederholt werden.

5. Methode nach Anspruch 2, wobei (b) - (d) mindestens 20-mal wiederholt werden.

6. Methode nach Anspruch 2, wobei (b) - (d) mindestens 50-mal wiederholt werden.

7. Methode nach Anspruch 1, wobei das Regenerierungspuffermittel Natriumthiosulfat umfasst.

8. Methode nach Anspruch 1, wobei das Regenerierungspuffermittel Peressigsäure umfasst.

9. Methode nach Anspruch 2, wobei die aus Blut gewonnene Zusammensetzung aus der Gruppe ausgewählt wird bestehend aus Vollblut, Plasma, Plättchenzubereitungen, frischem gefrorenem Plasma, Lösungsmittel-Detergens-Plasma, IVIG, IgM-Zubereitungen, gereinigten Koagulationsfaktorkonzentraten und Fibrinogenkonzentrate.

10. Methode nach Anspruch 2, wobei durch den Trennungsschritt Blutzusammensetzungsproteine aus dem zweiten Antigen-Isoagglutinin-Komplex entfernt werden.

11. Methode nach Anspruch 10, wobei die Proteine auf Blutbasis mindestens einen der Blutkoagulationsfaktoren: Faktor V, Faktor VII, Faktor VIII, Faktor IX, Faktor X, Faktor XI, Faktor XII und Faktor XIII; Antikörper, bei denen es sich nicht um A- und B-Isoagglutinine handelt; angeborene Proteine und Enzyme; von Willebrand-Faktor-Metalloproteinase und Kallikrein enthalten.

12. Methode nach Anspruch 1, wobei das Antigen ein oder mehrere Trisaccharide umfasst.

13. Methode für das selektive Entfernen eines Isoagglutinins aus einer aus Blut gewonnenen Zusammensetzung, wobei das Methode Folgendes umfasst:
(a) Bereitstellen eines Harzes, das an ein für das Isoagglutinin spezifisches Antigen angeknüpft ist;
(b) Waschen des Harzes mit einem Regenerierungspuffermittel unter Bildung eines regenerierten Harzes;
(c) Kontaktieren des gewaschenen Harzes mit der aus Blut gewonnenen Zusammensetzung unter Bedingungen, die die Bildung eines Isoagglutinin-Antigen-Komplexes auf dem Harz erlauben;
(d) Abtrennen der aus Blut gewonnenen Zusammensetzung von dem Isoagglutinin-Antigen-Komplex; und
(e) Wiederholen der Schritte (b) - (d) unter selektivem Entfernen des Isoagglutinins von der aus Blut gewonnenen Zusammensetzung.

14. Methode nach Anspruch 13, wobei die Schritte (b) bis (d) mindestens fünfmal wiederholt werden.

15. Methode nach Anspruch 13, wobei die Schritte (b) bis (d) mindestens zehnmal wiederholt werden.

16. Methode nach Anspruch 13, wobei die Schritte (b) bis (d) mindestens 25-mal wiederholt werden.

17. Methode nach Anspruch 13, wobei die Schritte (b) bis (d) mindestens 50-mal wiederholt werden.

18. Methode nach Anspruch 13, wobei die Schritte (b) bis (d) mindestens 75-mal wiederholt werden.

19. Methode nach Anspruch 13, wobei die Schritte (b) bis (d) mindestens 100-mal wiederholt werden.

20. Methode nach Anspruch 13, wobei das Regenerierungspuffermittel Natriumthiosulfat umfasst.

21. Methode nach Anspruch 20, wobei das Regenerierungspuffermittel 3M-Natriumthiosulfat umfasst.

22. Methode nach Anspruch 13, wobei das Regenerierungspuffermittel Peressigsäure umfasst.

23. Methode nach Anspruch 13, wobei das Kontaktieren durch Säulenchromatographie stattfindet.

24. Methode nach Anspruch 13, wobei das Kontaktieren durch chargenweise Absorption stattfindet.

25. Methode nach Anspruch 1 und Anspruch 13, wobei das Waschen ein Virus oder Prion auf dem Harz inaktiviert, nicht infektiös macht oder entfernt.

26. Methode nach Anspruch 1 und Anspruch 13, wobei es sich bei dem Harz um Toyopearl AF-Carboxypolymethacrylatharz handelt.

27. Methode für das selektive Entfernen eines Isoagglutinins aus einer aus Blut gewonnenen Zusammensetzung, wobei das Methode Folgendes umfasst:
(a) Bereitstellen eines Toyopearl-AF-Carboxypolymethacrylatharzes, das an ein für das Isoagglutinin spezifisches Antigen angeknüpft ist;
(b) Waschen des Harzes mit Natriumthiosulfat unter Bildung eines regenerierten Harzes;
(c) Kontaktieren des gewaschenen Harzes mit der aus Blut gewonnenen Zusammensetzung unter Bedingungen, die die Bildung eines Isoagglutinin-Antigen-Komplexes auf dem Harz erlauben;
(d) Abtrennen der aus Blut gewonnenen Zusammensetzung von dem Isoagglutinin-Antigen-Komplex; und
(e) Wiederholen der Schritte (b) - (d) unter selektivem Entfernen des Isoagglutinins von der aus Blut gewonnenen Zusammensetzung, während andere Blutproteine in der aus Blut gewonnenen Zusammensetzung zurückgehalten werden.

28. Methode nach Anspruch 27, wobei Schritt (b) des Weiteren das Waschen des Harzes mit Peressigsäure umfasst.

29. Methode nach Anspruch 1, Anspruch 13 und Anspruch 27, wobei es sich bei Isoagglutinin um Anti-A-Isoagglutinin, Anti-B-Isoagglutinin oder Anti-A,B-Isoagglutinin handelt.

30. Zusammensetzung für das Binden eines Isoagglutinins, wobei die Zusammensetzung ein Polymethacrylatharz umfasst, das an ein für das Isoagglutinin spezifisches Antigen angeknüpft ist, wobei das Antigen durch eine Carboxylgruppe auf dem Harz an das Harz angeknüpft ist.

31. Zusammensetzung nach Anspruch 30, wobei es sich bei dem Antigen um ein Oligosaccharid handelt.

32. Zusammensetzung nach Anspruch 31, wobei es sich bei dem Antigen um ein Trisaccharid handelt.

## Revendications

1. Procédé de régénération d'une résine pour éliminer une isoagglutinine d'une composition dérivée du sang, le procédé comprenant :
(a) la mise à disposition d'une résine liée à un complexe antigène-isoagglutinine ; et
(b) le lavage de la résine à l'aide d'un tampon de régénération, dans des conditions suffisantes à éliminer sélectivement l'isoagglutinine du complexe antigène-isoagglutinine, en régénérant ainsi la résine.

2. Procédé selon la revendication 1, comprenant en sus :
(c) la mise en contact de la résine régénérée avec une composition dérivée du sang, comprenant ladite isoagglutinine, dans des conditions suffisantes à former un deuxième complexe antigène-isoagglutinine ; et
(d) la séparation de la composition dérivée du sang du deuxième complexe antigène-isoagglutinine, en séparant ainsi la composition dérivée du sang de l'isoagglutinine.

3. Procédé selon la revendication 2, dans lequel les étapes (b)-(d) sont répétées à au moins deux reprises.

4. Procédé selon la revendication 2, dans lequel les étapes (b)-(d) sont répétées à au moins 5 reprises.

5. Procédé selon la revendication 2, dans lequel les étapes (b)-(d) sont répétées à au moins 20 reprises.

6. Procédé selon la revendication 2, dans lequel les étapes (b)-(d sont répétées à au moins 50 reprises.

7. Procédé selon la revendication 1, dans lequel le tampon de régénération comprend du thiosulfate de sodium.

8. Procédé selon la revendication 1, dans lequel le tampon de régénération comprend de l'acide peracétique.

9. Procédé selon la revendication 2, dans lequel la composition dérivée du sang est sélectionnée parmi le groupe constitué du sang entier, du plasma, de préparations de plaquettes, de plasma fraîchement congelé, de plasma détergent-solvant, de IVIG, de préparations IgM, de concentrés de facteurs de coagulation purifiés et de concentrés de fibrinogène.

10. Procédé selon la revendication 2, dans lequel l'étape de séparation élimine les protéines à base de sang du deuxième complexe antigène-isoagglutinine.

11. Procédé selon la revendication 10, dans lequel les protéines à base de sang comprennent au moins l'un des facteurs de coagulation du sang : les facteurs V, VII, VIII, IX, X, XI, XII et XIII ; des anticorps autres que les isoagglutinines A et B ; les protéines et des enzymes latentes ; la métalloprotéinase de facteur von Willebrand, et la kallicréine.

12. Procédé selon la revendication 1, dans lequel l'antigène comprend un ou plusieurs tri saccharides.

13. Procédé pour éliminer sélectivement une isoagglutinine d'une composition dérivée du sang, le procédé comprenant :
(a) la mise à disposition d'une résine liée à un antigène spécifique pour l'isoagglutinine ;
(b) le lavage de la résine à l'aide d'un tampon de régénération pour former une résine régénérée ;
(c) la mise en contact de la résine lavée avec la composition dérivée du sang dans des conditions permettant la formation d'un complexe antigène-isoagglutinine sur la résine ;
(d) la séparation de la composition dérivée du sang du complexe antigène-isoagglutinine ; et
(e) la répétition des étapes (b)-(d), en éliminant ainsi sélectivement l'isoagglutinine de la composition dérivée du sang.

14. Procédé selon la revendication 13, dans lequel les étapes (b) à (d) sont répétées à au moins cinq reprises.

15. Procédé selon la revendication 13, dans lequel les étapes (b) à (d) sont répétées à au moins dix reprises.

16. Procédé selon la revendication 13, dans lequel les étapes (b) à (d) sont répétées à au moins 25 reprises.

17. Procédé selon la revendication 13, dans lequel les étapes (b) à (d) sont répétées à au moins 50 reprises.

18. Procédé selon la revendication 13, dans lequel les étapes (b) à (d) sont répétées à au moins 75 reprises.

19. Procédé selon la revendication 13, dans lequel les étapes (b) à (d) sont répétées à au moins 100 reprises.

20. Procédé selon la revendication 13, dans lequel le tampon de régénération comprend du thiosulfate de sodium.

21. Procédé selon la revendication 20, dans lequel le tampon de régénération comprend 3M de thiosulfate de sodium.

22. Procédé selon la revendication 13, dans lequel le tampon de régénération comprend de l'acide peracétique.

23. Procédé selon la revendication 13, dans lequel la mise en contact est effectuée par chromatographie sur colonne.

24. Procédé selon la revendication 13, dans lequel la mise en contact est effectuée par absorption par lots.

25. Procédé selon la revendication 1 et selon la revendication 13, dans lequel le lavage inactive, rend non infectieux ou élimine un virus ou un prion sur la résine.

26. Procédé selon la revendication 1 et la revendication 13, dans lequel la résine est une résine de carboxypolyméthacrylate Toyopearl AF.

27. Procédé pour éliminer sélectivement une isoagglutinine d'une composition dérivée du sang, le procédé comprenant :
(a) la mise à disposition d'une résine de carboxypolyméthacrylate Toyopearl AF, liée à un antigène spécifique pour l'isoagglutinine ;
(b) le lavage de la résine à l'aide de thiosulfate de sodium pour former une résine régénérée ;
(c) la mise en contact de la résine lavée avec la composition dérivée du sang dans des conditions permettant la formation d'un complexe antigène-isoagglutinine sur la résine ;
(d) la séparation de la composition dérivée du sang du complexe antigène-isoagglutinine ; et
(e) la répétition des étapes (b)-(d), en éliminant ainsi sélectivement l'isoagglutinine de la composition dérivé du sang, tout en retenant les autres protéines sanguines dans la composition dérivée du sang.

28. Procédé selon la revendication 27, dans lequel l'étape (b) comprend en outre le lavage de la résine avec de l'acide peracétique.

29. Procédé selon la revendication 1, selon la revendication 13 et selon la revendication 27, dans lequel l'isoagglutinine est l'isoagglutinine anti-A, l'isoagglutinine anti-B ou l'isoagglutinine anti-A,B.

30. Composition en vue de la liaison d'une isoagglutinine, la composition comprenant une résine de polyméthacrylate liée à un antigène spécifique pour ladite isoagglutinine, dans laquelle ledit antigène est lié à ladite résine par un groupement carboxyle sur ladite résine.

31. Composition selon la revendication 30, dans laquelle ledit antigène est un oligo saccharide.

32. Composition selon la revendication 31, dans laquelle ledit antigène est un tri saccharide.
